# EUROPEAN PATENT SPECIFICATION

(11) **EP 1 673 037 B1**
(45) Date of publication and mention of the grant of the patent: **26.08.2009**
(21) Application number: 04794695.9
(22) Date of filing: 08.10.2004
(51) Int. Cl.: A61F 2/06

(54) **ENDOLUMINAL PROSTHESIS WITH INTERCONNECTABLE MODULES**
ENDOLUMINALE PROTHESE MIT VERBINDBAREN MODULEN
PROTHESE ENDOLUMINALE A MODULES INTERCONNECTABLES

(30) Priority: 10.10.2003 US 510617 P
(43) Date of publication of application: 28.06.2006
(73) Proprietor: THE CLEVELAND CLINIC FOUNDATION, Cleveland, OH 44195 (US)
(72) Inventor: GREENBERG, Roy, K., Bratenahl, OH 44108 (US); WEST, Karl, Geneva, OH 44041 (US)
(74) Representative: Jehan, Robert
(86) International application number: PCT/US2004/033420
(87) International publication number: WO 2005/034803

(56) References cited:
- EP-A- 1 266 635
- WO-A-03/075798
- WO-A-20/05027784
- US-A- 6 110 198
- US-B1- 6 325 823

## Description

This invention relates to a medical device and, in particular, a prosthesis for implantation within the human or animal body for the repair of damaged vessels such as blood vessels.

### BACKGROUND

Throughout this specification, when discussing the application of this invention to the aorta or other blood vessels, the term "distal" with respect to an abdominal device is intended to refer to a location that is, or a portion of the device that when implanted is, further downstream with respect to blood flow; the term "distally" means in the direction of blood flow or further downstream.

The term proximal" is intended to refer to a location that is, or a portion of the device that when implanted is, further upstream with respect to blood flow; the term "proximally" means in the direction opposite to the direction of blood flow or further upstream.

The functional vessels of human and animal bodies, such as blood vessels and ducts, occasionally weaken or even rupture. For example, the aortic wall can weaken, resulting in an aneurysm. Upon further exposure to hemodynamic forces, such an aneurysm can rupture. In Western European and Australian men who are between 60 and 75 years of age, aortic aneurysms greater than 29mm in diameter are found in 6.9% of the population, and those greater than 40mm are present in 1.8% of the population.

A common surgical intervention for weakened, aneurismal, dissected or ruptured vessels is the use of a prosthesis to provide some or all of the functionality of the original, healthy vessel and/or preserve any remaining vascular integrity by replacing a length of the existing vessel wall that spans the site of vessel failure.

It is recommended that these prostheses seal off the failed portion of the vessel. For weakened or aneurismal vessels, even a small leak can be lead to the pressurization of or flow in the original vessel, which aggravates the condition the prosthesis was intended to treat. A prosthesis of this type can, for example, treat aneurysms of the thoracic aortic, abdominal aortic, aortoiliac, iliac, or branch vessels.

It prosthesis can be of a unitary construction, or be comprised of multiple prosthetic modules. A modular prosthesis allows a surgeon to accommodate a wide variation in vessel morphology while reducing the necessary inventory of differently sized prostheses.

For example, aortas are quite variable in length, diameter and angulation between the renal artery region and the region of the aortic bifurcation. Prosthetic modules that fit each of these variables can be assembled to form a prosthesis, obviating the need for a custom prosthesis or large inventories of prostheses that accommodate all possible combinations of these variables. A modular system may also improve deployment characteristics by allowing the proper placement of one module before the implantation of an adjoining module.

Modular systems are typically assembled *in situ* by overlapping the ends of the prosthetic modules so that the end of one module sits partially inside the other module to form a circumferential apposition. This attachment process is called telescoping.

Modular prostheses are known for use in treating descending thoracic and abdominal aortic aneurysms, where the prosthesis at the proximal end defines a single lumen for placement within the aorta and at the other end is bifurcated for extension into the iliac arteries. Iliac extension modules can be connected to the ends of the bifurcation.

One such modular system, disclosed in PCT application WO 98/53761, is an endoluminal prosthesis which is, in particular, useful for repair of aortic aneurysms. This application discloses a prosthesis which includes a sleeve or tube of biocompatible prosthesis material such as woven polyester fabric or polytetrafluoroethylene (PTFE) defining a lumen, and further includes several stents secured therealong. The prosthesis is designed to span an aneurysm that extends along the aorta proximally from the two iliac arteries. This reference also discloses the manner of deploying the stent prosthesis in the patient utilizing an introducer assembly.

In the WO 98/53761 application, the material-covered portion of the single-lumen proximal end of the prosthesis bears against the wall of the aorta above the aneurysm to seal off the aneurysm at a location that is spaced distally of the entrances to the renal arteries. Thin wire struts of a proximal stent traverse the renal artery entrances without occluding them, since no prosthesis material is utilized along the proximal stent while securing the stent prosthesis in position within the aorta when the stent self-expands.

An extension module is affixed to one of the legs of the prosthesis to extend along a respective iliac artery and, optionally, extensions may be affixed to both legs. These extension modules are attached by telescoping. The deployment of a modular endoluminal prosthesis into the lumen of a patient from a remote location by the use of a deployment device or introducer is disclosed in the same patent application.

One modular prosthesis approved by the Food and Drug Administration (FDA) to treat aortic aneurysms is the ZENITH^{®} AAA Endovascular Graft sold by Cook Incorporated. The ZENITH^{®} AAA Endovascular Graft may be made up of three prosthetic modules: a main body module and two leg modules. The main body is positioned in the aorta. The legs are positioned in the iliac arteries and connect to the main body. The prosthesis thus extends from the aorta below the renal arteries into both iliac arteries. The prosthesis itself is made of a woven polyester material like that used in open surgical repair. Standard surgical suturing techniques are used to sew the graft material to a frame of stainless steel stents. These self-expanding stents provide support for the graft material. Other modular prostheses are described in U.S. Patent No. 6,325,823; U.S. Patent No. 6,110,198; WO 03/075798; and EP Patent No. 1,266,635.

The connections between prosthetic modules are typically maintained by friction fit, radial force or other locking mechanism that relies on the circumferential apposition of the two prosthetic modules. The friction fit can be enhanced by the radial force exerted by the internal prosthetic module on the external prosthetic modules where the two overlap, and be further enhanced by stents fixed to the modules at the overlap region.

In some modular systems, hemodynamic forces or other forces may tend to compromise the integrity of the intermodular seal or the modules can become fully or partially dislocated. Modular disconnections may also result, at least in part, from morphologic changes in the vessel. Such a compromise could result in an endoleak, which prevents the prosthesis from performing its function of excluding a length of the original vessel. An endoleak, even a relatively small one, resulting from intermodular pull-out or endoleak will result in the aneurysm repressurizing. A repressurized aneurysm has a tendency to rupture-an event associated with an extremely high mortality rate.
US 6,325,823 describes a prosthesis comprising a plurality of telescoping tubular members that are deployed and assembled in vivo to define a lumen through a diseased portion of a vascular system. The individual tubular members are capable of accommodating torsional and longitudinal displacements caused by relative motion of the healthy portions on either side of the diseased portion of vessel.
WO 2005/027784 describes a system and method for treating and repairing complex anatomy characterized by a plurality of vessel portions oriented at various angles relative to each other. The system including a graft device that is capable of being assembled in situ and has associated therewith a method that avoids the cessation of blood flow to vital organs. A delivery catheter system and various graft supporting, mating and anchoring structures are additionally included. This document is prior art under Article 54(3) EPC only.

There is thus a need for an improved design for the interconnection between prosthetic modules which reduces leaks by preventing modular dislocation.

### BRIEF SUMMARY

In one aspect of the invention, there is a modular endoluminal prosthesis which comprises a first prosthetic module having an opening at one end and an internal surface, and a second prosthetic module having an opening at one end and an external surface, wherein the first and second modules are connectable by inserting the one end of the second module into the one end of the first module, and wherein either the internal surface or the external surface comprises at least one projection and wherein the other of the internal surface or the external surface comprises at least one surface feature, said projection and said surface feature being at least partially complementary so that surface feature engages the projection when the modules are connected.

The modular endoluminal prosthesis may also comprise a telescoping interconnection between the first prosthetic module and the second prosthetic module, wherein the telescoping interconnection is capable of resisting a pull-out force of greater than 4 Newtons.

The first prosthetic module may have an internal surface at one end comprising circumferential ridges and the second prosthetic module may have an external surface at one end comprising circumferential ridges, at least some of which engage at least some of the circumferential ridges of the internal surface when the one end of the second prosthetic module is inserted into the one end of the first prosthetic module, to thereby provide an interlocking connection between the first prosthetic module and the second prosthetic module.

This then generally describes the invention, but to assist with understanding, reference will now be made to the accompanying drawings which show preferred embodiments of the invention.

### BRIEF DESCRIPTION OF THE DRAWINGS

FIG. 1A shows a front perspective view of a prosthetic module with a helical crimp;

FIG. 1B shows a front perspective view of a prosthetic module with annular crimps;

FIG. 2 shows a front perspective view of a prosthetic module with parallel helical crimps;

FIG. 3 shows a front perspective view of a prosthetic module with a single dimple;

FIG. 4A shows a front perspective view of two interconnectable but separate prosthetic modules;

FIG. 4B shows a front perspective view of the prosthetic modules of FIG. 4A, following telescoping interconnection;

FIG. 5A shows a schematic cross-sectional representation of the prosthetic modules of FIG. 4B;

FIG. 5B shows a schematic cross-sectional representation of interconnected prosthetic modules which have an engagement region shorter than the overlap region;

FIG. 6A shows a partial sectional view of two interconnected prosthetic modules, each with stents attached;

FIG. 6B shows a front perspective view of the external prosthetic module of FIG. 6A;

FIG. 7 shows an exploded perspective view of an embodiment of an introducer in perspective view with a partially deployed, bifurcated aortic prosthesis;

FIG. 8A shows a frontal, partial cross-sectional view of prosthetic modules deployed in the aortic and renal arteries;

FIG. 8B shows a frontal, partial cross-sectional view of an embodiment in the aortic and illac arteries;

FIG. 8C shows a frontal, partial cross-sectional view of an embodiment in the iliac and hypogastric arteries;

FIG. 8D shows a frontal, partial cross-sectional view of an embodiment deployed in the abdominal aorta and having a helical prosthetic branch;

FIG. 9A shows a frontal, partial cross-sectional view of an embodiment in the thoracic aortic artery;

FIG. 9B shows a frontal, partial cross-sectional view of an embodiment in the thoracic aortic and left subclavian arteries; and

FIG. 9C shows a frontal, partial cross-sectional view of an embodiment having a helical prosthetic branch and deployed into the thoracic aorta.

### DETAILED DESCRIPTION

The present invention relates to a modular endoluminal prosthesis that is implanted in a vessel or lumen in order to exclude damaged vascular tissue and/or preserve at least some of the existing vascular function. The present invention also relates to minimizing, if not eliminating, intermodular pull-out and leaks.

The component prosthetic modules are connected by telescoping. Telescoping describes the process of establishing the circumferential overlap of two tubular grafts by fitting the end of one tubular graft into the other end The two ends are preferably of nearly the same diameter so that circumferential apposition can be promoted through the overlap region.

The telescoping connections between the prosthetic modules can be significantly and advantageously improved by having specific surface geometries or features on the contact surfaces of those prosthetic modules. There are preferably specific contact surface geometries on one prosthetic module which engage similar contact surface geometries on the interconnected prosthetic module. This may decrease the risk of dislocation.

The term "prosthesis" means any replacement for a body part or function of that body part It can also mean a device that enhances or adds functionality to a physiological system,

The term "endoluminal" describes objects that are found or can be placed inside a lumen in the human or animal body. A lumen can be an existing lumens or a lumen created by surgical intervention. This includes lumens such as blood vessels, parts of the gastrointestinal tract, ducts such as bile ducts, parts of the respiratory system, etc. "Endoluminal prosthesis" thus describes a prosthesis that can be placed inside one of these lumens.

The term "graft" means the generally cannular or tubular member which acts as an artificial vessel. A graft by itself, or with the addition of stents and/or other elements, can be an endoluminal prosthesis.

The term "stent" means any device or structure that adds rigidity, expansion force or support to a graft or prosthesis.

The term "pull-out force" means the maximum force of resistance to partial or full dislocation provided by a modular prosthesis. The pull-out force of a prosthesis having two interconnected modules can be measured by an MTS ALLIANCE RT/5^{®} tensile testing machine, which is available from the MTS Corporation, Eden Prairie, Minnesota, USA. The MTS machine is connected to a computer terminal that is used to control the machine, collect, and process the data. A pressurization pump system is attached to the load cell located on the tensile arm of the MTS machine. One end of the prosthesis is connected to the pressurization pump, which provides an internal pressure of 60mmHg to simulate the pressure exerted by blood upon the device when deployed *in vivo.* The other end of the prosthesis is sealed. The prosthesis is completely immersed in a 37°C water bath during the testing to simulate mean human body temperature. The MTS machine pulls the devices at 0.1mm increments until the devices are completely separated. The computer will record, *inter alia,* the highest force with which the modules resist separation, *i.e*. the pull-out force.

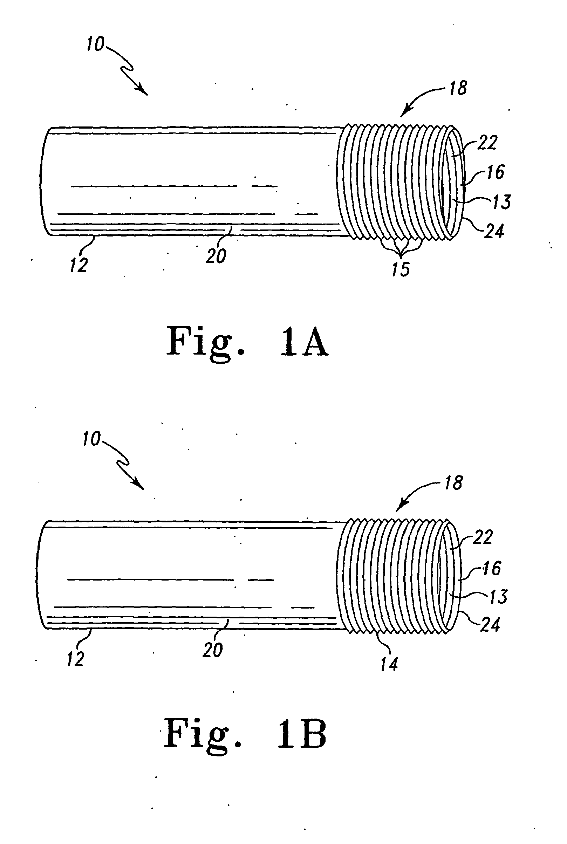

The embodiment of the present invention shown in Figure 1A is a prosthetic module 10, which includes a substantially tubular graft 12 having a passageway or lumen 13 extending therethrough. The tubular graft 12 has an external surface 20, an internal surface 22 and a terminus 24. The purpose of the tubular graft 12 is to contain and/or shunt blood or other fluid.

The size and shape of the prosthetic module 10 can vary significantly depending on the anatomy in which it is to be implanted, and the corresponding module to which this prosthetic module 10 will be connected. For example, the tubular graft 12 can have a taper, turns or any other suitable geometry.
Physiological variables, deployment characteristics and other factors contribute to the determination of proper size and shape of the prosthetic module 10.

The tubular graft material is preferably non-porous so that it does not leak or sweat under physiologic forces. The graft material is preferably made of woven or knitted polyester (Vascutek Ltd., Renfrewshire, Scotland, UK). Other biocompatible fabrics, non-woven materials and porous sheets may be used as the graft material. Examples of biocompatible polymers from which porous sheets can be formed include polyesters, such as poly(ethylene terephthalate), polylactide, polyglycolide and copolymers thereof; fluorinated polymers, such as PTFE, expanded PTFE and poly(vinylidene fluoride); polysiloxanes, including polydimethyl siloxane; and polyurethanes, including polyetherurethanes, polyurethane ureas, polyetherurethane ureas, polyurethanes containing carbonate linkages and polyurethanes containing siloxane segments. In addition, materials that are not inherently biocompatible may be subjected to surface modifications in order to render the materials biocompatible. Examples of surface modifications include graft polymerization of biocompatible polymers from the material surface, coating of the surface with a crosslinked biocompatible polymer, chemical modification with biocompatible functional groups, and immobilization of a compatibilizing agent such as heparin or other substances. Thus, any polymer that may be formed into a porous sheet can be used to make a graft material, provided the final porous material is biocompatible. Polymers that can be formed into a porous sheet include polyolefins, polyacrylonitrile, nylons, polyaramids and polysulfones, in addition to polyesters, fluorinated polymers, polysiloxanes and polyurethanes as listed above. Preferably the porous sheet is made of one or more polymers that do not require treatment or modification to be biocompatible.

The graft material may include a biocompatible polyurethane. Examples of biocompatible polyurethanes include THORALON^{®} (Thoratec, Pleasanton, CA),BIOSPAN^{®}, BIONATE^{®}**,** ELASTHANE^{™}, PURSIL^{™} and CARBOSIL^{™} (Polymer Technology Group, Berkeley, CA). As described in U. S. Patent Application Publication No. 2002/0065552 Al, THORALON^{®} is a polyetherurethane urea blended with a siloxane- containing surface modifying additive. Specifically, the polymer is a mixture of base polymer BPS-215 and an additive SMA-300.

The graft material may also include extracellular matrix materials. The "extracellular matrix" is a collagen-rich substance that is found in between cells in animal tissue and serves as a structural element in tissues. It is typically a complex mixture of polysaccharides and proteins secreted by cells. The extracellular matrix can be isolated and treated in a variety of ways. Following isolation and treatment, it is referred to as an "extracellular matrix material," or ECMM. ECMMs may be isolated from submucosa (including small intestine submucosa), stomach submucosa, urinary bladder submucosa, tissue mucosa, renal capsule, dura mater, liver basement membrane, pericardium or other tissues.

Purified tela submucosa, a preferred type of ECMM, has been previously described in U. S. Patent Nos. 6,206, 931,6, 358, 284 and 6,666, 892 as a biocompatible, n on-thrombogenic material that enhances the repair of damaged or diseased host tissues. Purified submucosa extracted from the small intestine ("small intestine submucosa" or "SIS") is a more preferred type of ECMM for use in this invention. Another type of ECMM, isolated from liver basement membrane, is described in U.S. Patent No. 6,379,710. ECMM may also be isolated from pericardium, as described in U. S. Patent No. 4,502, 159. Irrespective of the origin of the graft material, the graft material can be made thicker by making multi-laminate constructs, for example SIS constructs as described in U. S. Patent Nos. 5,968,096; 5,955,110; 5,885,619; and 5,711,969.

Projections 18 in the embodiment shown in Figure 1A extend from the terminus 24 to cover a length of the tubular graft 12. The projections 18 do not necessarily cover an entire circumference of the tubular graft 12, nor do they have to extend to the terminus 24.

The term "projection" means any element or unit of surface relief, such as a crimp, corrugation, ridge or dimple. The terms "projection" and "surface feature" exclude the roughness that exists solely in the planar weave of a textile, fabric or similar surface. "Projection" also includes a single circumvolution (360- degree turn) of a continuous helical or annular projection and any surface relief that projects from the internal surface of the tubular graft 12.

A "surface feature" is an element or unit of surface geometry (i.e., a projection or groove, or the like) that is capable of engaging a projection when the surface feature contacts the projection. Projections and surface features can be closely related. For example, a crimped surface has both surface features and projections. Therefore, for example, reference can be made to projections engaging surface features, or projections engaging projections. A "ridge" is a category of projections or surface features that include crimps, corrugations, and other surface geometries.

The projections 18 on the tubular graft 12 are the result of a specific surface geometry modification. In the embodiment shown in Figure 1A, the projections 18 are in the shape of a helical crimp or ridge in the fabric of the tubular graft 12. Thus, there are helical crimp or ridge peaks 15 on the external surface 20 of the tubular graft 12, and corresponding, complementary surface features 16 on the internal surface 22 of the tubular graft 12. Therefore, this particular tubular graft 12 has both projections 18 and surface features on both the internal surface 22 and the external surface 20.

In a preferred embodiment, the helical crimp like that shown in Figure 1A can be created by mounting the tubular graft 12 over a mandrel of substantially the same diameter. A thread, wire or other filament is wrapped helically around the tubular graft 12. The helical pitch is preferably between about 0.5mm and about 4mm and more preferably between about 1mm and about 3mm. The desired pitch can vary depending on the diameters of the interconnected modules, the characteristics of the tubular graft 12, and the desired pull-out force.

The assembly as described is then heated to a temperature of 138°C for eight (8) hours. Other temperatures can be used. Typically, the higher the temperature, the shorter the time required for adequate crimping, and vice versa. This may induce helical crimping in the wrapped portion of the prosthesis 10. This process can be varied to suit the constituent polymer used in the prosthesis 10, the thickness of the fabric or fiber, the thread count (if the fabric is woven), the desired rigidity of the crimping, and other factors.

The tubular graft 12 may also be molded, folded, mechanically crimped or corrugated, or treated in any way known to those of skill in the art to create projections and/or surface features. A ridge, including a corrugation, can also be woven into the fabric itself.

As shown in Figure 1B, parallel annular or ring-shaped ridges or crimps 14 can be employed. These projections and surface features can be created by wrapping multiple filaments in a circular fashion on a length of the tubular graft 12 and then heat treating the assembly using the method described above. The parallel rings can be spaced by any suitable distance, preferably between 0.5mm and 4mm and more preferably between 1 mm and 3mm.

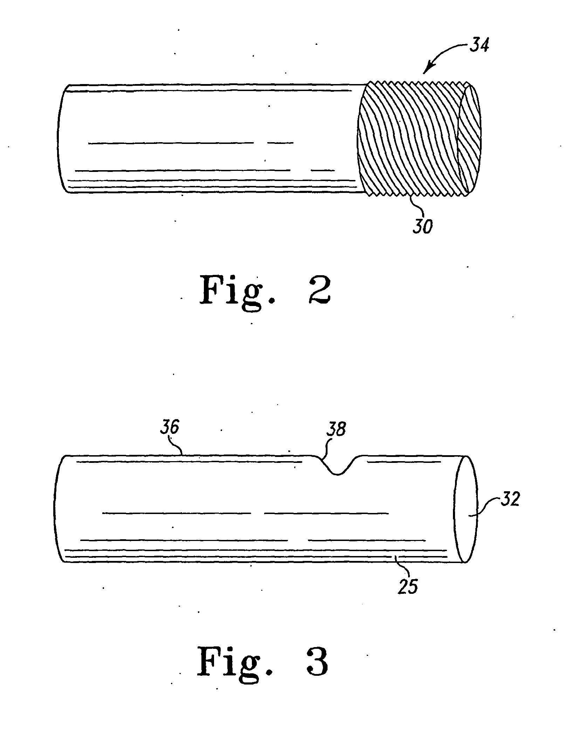

As shown in Figure 2, multiple parallel helices 34 instead of a single helix can be created. The process used to create a single helix, as described above in reference to Figure 1, is employed. However, multiple filaments, instead of a single filament, are wrapped in parallel helices over a length of the tubular graft 30. Any number of helices with any suitable pitch and any suitable angulation relative to the tubular graft 30 can be used. The pitch for multiple parallel helices is preferably between about 0.5mm and about 4mm and more preferably between about 1mm and about 3mm. The relative angulation is preferably between about 5 and about 10 degrees, and more preferably between about 5 and about 6 degrees. The desired pitch and angulation can vary depending on the diameters of the interconnected modules, the characteristics of the tubular graft.30, and the desired pull-out force.

As shown in Figure 3, the tubular graft 36 can also comprise a dimple 38 or dimples. Such a dimple is considered to create a projection on the internal surface 32 and a surface feature on the external surface 25 of the tubular graft 36. Such a graft 36 can interconnect with a graft with a similar dimple so that the dimples index or register with each other. Furthermore, different types of projections can be used together on a single prosthetic module.

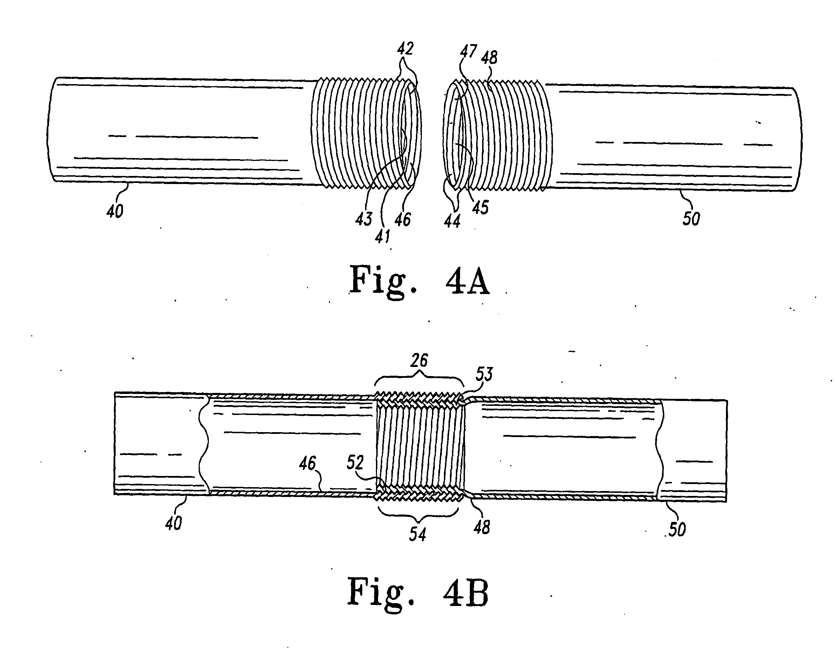

Figure 4A shows two prosthetic modules 40, 50 that are suitable for interconnection. The modules 40, 50 are approximately the same diameter. The first or external module 40 has an opening 41 at one end 43 and an internal surface 46. The second or internal prosthetic module 50 has an opening 45 at one end 47 and an external surface 48. A large difference in diameter can prevent circumferential apposition, thereby preventing adequate intermodular seal.

Preferably, the projections and surface feature 42,44 on both modules 40, 50 are substantially matching or complementary. This means that the projections and surface features 42, 44 have substantially the same frequency and amplitude or are otherwise similar. As shown in Figure 4A, the projections 42, 44 on both prosthetic modules 40, 50 are in the form of helical crimps which have approximately the same pitch.

Complementary projections tend to maximize the surface contact between opposing contact surfaces and ensure that the projections engage or index with opposing projections. Regardless of the type of projection employed, the pull-out force may be increased and the incidence of endoleaks may be decreased if there are substantially complementary projections.

The telescoping interconnection is formed by inserting one end 47 of the internal prosthetic module 50 into one end 43 of the external prosthetic module 40. Figure 4B shows the resulting interconnection of prosthetic modules 40, 50. The external prosthetic module 40 overlaps the internal prosthetic module 50, to form an overlap region 26.

Because these prosthetic modules 40, 50 are connected in this manner, the contact surface of the external prosthetic module 40 is the internal surface 46 and the contact surface of the internal prosthetic module 50 is the external surface 48. Therefore, the external projections 52 on the internal module 50 engage the internal projections 53 of the external module 40 to decrease the risk of modular dislocation and improve the seal so that the interconnection becomes more fluid-tight. The contact surfaces 46, 48 preferably have at least partially complementary projections 52, 53. Because the corresponding projections 52, 53 are complementary, they can substantially register or index with each other.

The overlap region 26 can be of any suitable length. Typically, the longer the overlap region 26, the greater the pull-out force. In a two-piece thoracic endoluminal prosthesis, for example, the overlap region can be about 8cm, although overlap regions may be as short as about 1mm.

The engagement region 54 is the region in which corresponding, and preferably complementary, projections 52, 53 contact each other. The overlap region 26 can be longer than or the same length as the engagement region 54.

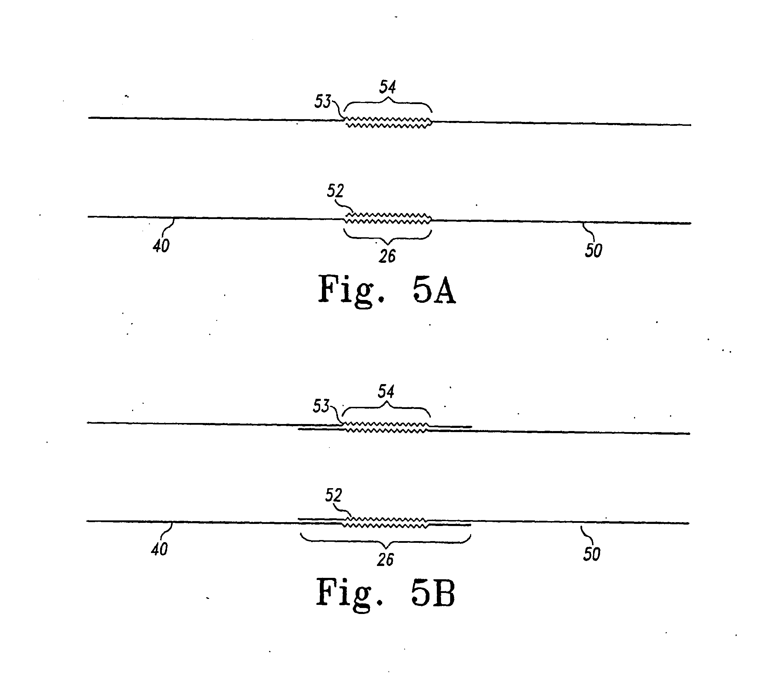

Figure 5A shows an engagement region 54 which is about the same length as the overlap region 26. Figure 5B shows interconnected modules in which the engagement region 54 is shorter than the overlap region 26.

In prosthetic modules where the projections do not extend through the entire overlap region or do not cover the entire circumference of the tubular graft, it is preferable to ensure that at least some of the projections on one module engage the complementary projections on the other module.

The projections or surface features 42, 44 preferably do not extend beyond the overlap region 26. This minimizes any unnecessary negative hemodynamic effects that may be caused by the projections or surface features 42, 44 that face the lumen. Projections which are adjacent to blood flow may have the effect of thickening the hemodynamic boundary layer, thereby impeding flow, and, in some cases, causing turbulence.

In Figure 5A, it is shown schematically how the corresponding, complementary projections 52, 53 engage one another. This encourages maximum surface contact between the complementary projections 52, 53. It is not necessary, however, for the complementary projections 52, 53 to perfectly index with each other.

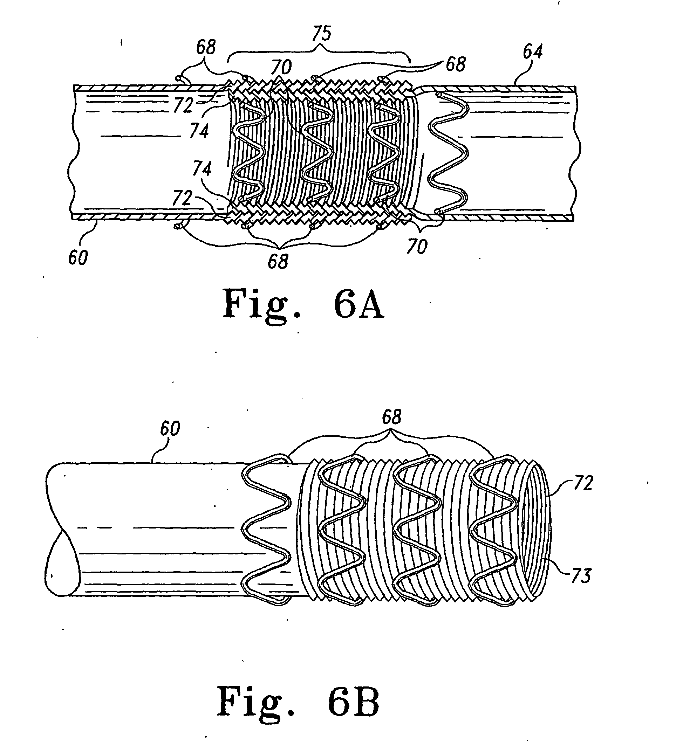

Figure 6A shows the use of stents to reinforce the modular interconnection. As shown in Figure 6A, it is preferable to employ one or more stents 68, 70 at the overlap region 75. Internal stents 70 may be attached to the internal module 64, while external stents 68 may be attached to the external module 60. This ensures that the stents 68, 70 reinforce the seal, without interfering with the engagement fit between the opposing projections 72, 74. The internal stents 70 are typically held at less than their relaxed diameter by the combined resisting force of the overlapping modules 60, 64 and the external stents 68. Internal stents fixed to the internal surface of the internal prosthetic module can also be used without any opposing stents. Stents that are outside of the overlap region 75 will not interfere with the seal, and can therefore be attached to the internal or external surface of the prosthetic modules 60, 64.

Figure 6B shows an external prosthetic module 60 having projections 72 on the internal contact surface 73 and attached external stents 68. Any stent known in the art can be employed, preferably self-expanding zigzag stents. The Gianturco Z-stents commercially available from Cook Incorporated, Bloomington, Indiana can be used. The stents can be made ofnitinol or stainless steel. They can be self-expanding, balloon-expandable or shape memory stents. These stents can be attached to the external or internal surface of the tubular graft.

A stent can be attached with a single suture at the peak of each acute bend in the stent or, preferably, multiple sutures at each peak. The suture material can be Prolene (5-0) or any other material known to one of skill in the art. Any method of attaching stents known to those of skill in the art can be used.

### THE INTRODUCER

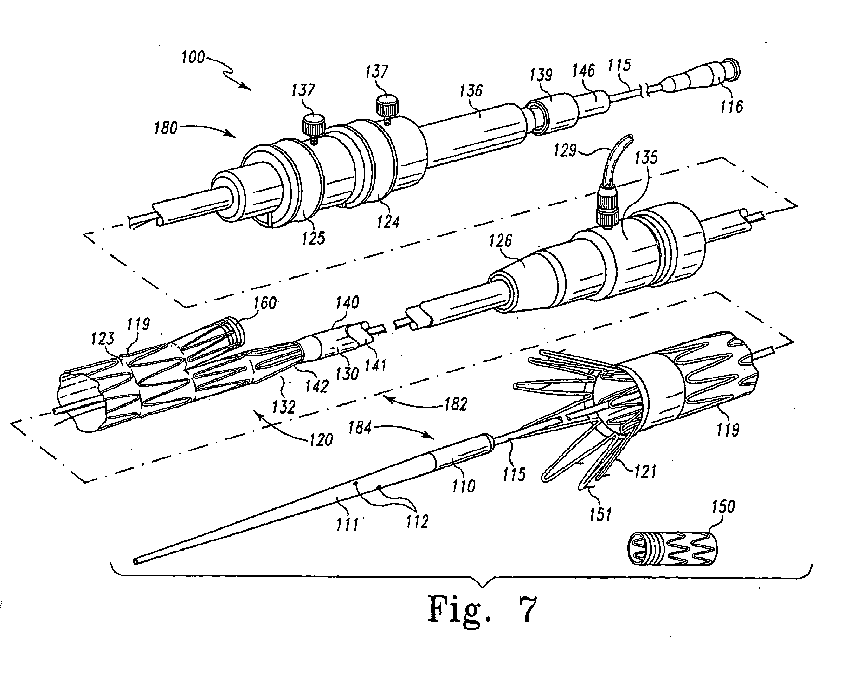

Figure 7 shows a self expanding bifurcated prosthesis 120, a self-expanding tubular prosthesis 150 (product code TFB 1 through TFB5, available from Cook Incorporated, Bloomington, Indiana) and an endovascular deployment system 100, also known as an introducer 100, for deploying the prosthesis 120 in a lumen of a patient during a medical procedure. These items are each described in greater detail in PCT application WO 98/53761.

The bifurcated prosthesis 120 has a generally inverted Y-shaped configuration. The prosthesis 120 includes a body 123, a shorter leg 160 and a longer leg 132. The bifurcated prosthesis 120 comprises a tubular graft material, such as woven polyester, with self-expanding stents attached thereto. The self-expanding stents 119 cause the prosthesis 120 to expand following its release from the introducer 100. The prosthesis 120 also includes a self-expanding zigzag stent 121 that extends from its proximal end. The self-expanding zigzag stent 121 has distally extending barbs 151. When it is released from the introducer 100, the self-expanding zigzag stent 121 anchors the barbs 151, and thus the proximal end of the prosthesis 120, to the lumen of the patient.

The self-expanding tubular prosthesis 150 is similar to the bifurcated prosthesis 120, but has a unitary (*i.e*., non-bifurcated) lumen. The tubular prosthesis 150 also comprises a tubular graft material, such as woven polyester, having self-expanding stents attached thereto. The tubular prosthesis 150 is configured to form a telescoping interconnection to the shorter leg 160 of the bifurcated prosthesis 120, such that projections on each can engage each other.

The introducer 100 includes an external manipulation section 180, a distal attachment region 182 and a proximal attachment region 184. The distal attachment region 182 and the proximal attachment region 184 secure the distal and proximal ends of the prosthesis 120, respectively. During the medical procedure to deploy the prosthesis 120, the distal and proximal attachment regions 182 and 184 will travel through the lumen to a desired deployment site. The external manipulation section 180, which is acted upon by a user to manipulate the introducer, remains outside of the patient throughout the procedure.

The proximal attachment region 184 of the introducer 100 includes a cylindrical sleeve 110. The cylindrical sleeve 110 has a long tapered flexible extension 111 extending from its proximal end. The flexible extension 111 has an internal longitudinal apertures (not shown). This longitudinal aperture facilitates advancement of the tapered flexible extension 111 along an insertion wire (not shown). The longitudinal aperture also provides a channel for the introduction of medical reagents. For example, it may be desirable to supply a contrast agent to allow angiography to be performed during placement and deployment phases of the medical procedure.

A thin walled metal tube 115 is fastened to the extension 111. The thin walled metal tube 115 is flexible so that the introducer 100 can be advanced along a relatively tortuous vessel, such as a femoral artery, and so that the distal attachment region 182 can be longitudinally and rotationally manipulated. The thin walled metal tube 115 extends through the introducer 100 to the manipulation section 180, terminating at a connection means 116.

The connection means 116 is adapted to accept a syringe to facilitate the introduction of reagents into the thin walled metal tube 115. The thin walled metal tube 115 is in fluid communication with the apertures 112 of the flexible extension 111. Therefore, reagents introduced into connection means 116 will flow to and emanate from the apertures 112.

A plastic tube 141 is coaxial with and radially outside of the thin walled metal tube 115. The plastic tube 141 is "thick walled" - its wall is preferably several times thicker than that of the thin walled metal tube 115. A sheath 130 is coaxial with and radially outside of the plastic tube 141. The thick walled plastic tube 141 and the sheath 130 extend distally to the manipulation region 180.

During the placement phase of the medical procedure, the prosthesis 120 is retained in a compressed condition by the sheath 130. The sheath 130 extends distally to a gripping and hemostatic sealing means 135 of the external manipulation section 180. During assembly of the introducer 100, the sheath 130 is advanced over the cylindrical sleeve 110 of the proximal attachment region 184 while the prosthesis 120 is held in a compressed state by an external force. A distal attachment (retention) section 140 is coupled to the thick walled plastic tube 141. The distal attachment section 140 retains a distal end 142 of the prosthesis 120 during the procedure. Likewise, the cylindrical sleeve 110 retains the self-expanding zigzag stent 121.

The distal end 142 of the prosthesis 120 is retained by the distal attachment section 140. The distal end 142 of the prosthesis 120 has a loop (not shown) through which a distal trigger wire (not shown) extends. The distal trigger wire extends through an aperture (not shown) in the distal attachment section 140 into an annular region between the thin walled tube 115 and the thick walled tube 141. The distal trigger wire extends through the annular space to the manipulation region 180. The distal trigger wire exits the annular space at a distal wire release mechanism 125.

The external manipulation section 180 includes a hemostatic sealing means 135. The hemostatic sealing means 135 includes a hemostatic seal (not shown) and a side tube 129. The hemostatic sealing means 135 also includes a clamping collar (not shown) that clamps the sheath 130 to the hemostatic seal, and a silicone seal ring (not shown) that forms a hemostatic seal around the thick walled plastic tube 141. The side tube 129 facilitates the introduction of medical reagents between the thick walled tube 141 and the sheath 130.

A proximal portion of the external manipulation section 180 includes a release wire actuation section that has a body 136. The body 136 is mounted onto the thick walled plastic tube 141. The thin walled tube 115 passes through the body 136. The distal wire release mechanism 125 and the proximal wire release mechanism 124 are mounted for slidable movement onto the body 136.

The positioning of the proximal and distal wire release mechanisms 124 and 125 is such that the proximal wire release mechanism 124 must be moved before the distal wire release mechanism 125 can be moved. Therefore, the distal end 142 of the prosthesis 120 cannot be released until the self-expanding zigzag stent 121 has been released, and the barbs 151 have been anchored to the lumen. Clamping screws 137 prevent inadvertent early release of the prosthesis 120. A hemostatic seal (not shown) is included so that the release wires can extend out through the body 136 without unnecessary blood loss during the medical procedure.

A distal portion of the external manipulation section 180 includes a pin vise 139. The pin vise 139 is mounted onto the distal end of the body 136. The pin vise 139 has a screw cap 146. When screwed in, vise jaws (not shown) of the pin vise 139 clamp against or engage the thin walled metal tube 115. When the vise jaws are engaged, the thin walled tube 115 can only move with the body 136, and hence the thin walled tube 115 can only move with the thick walled tube 141. With the screw cap 146 tightened, the entire assembly can be moved together as one piece.

A second introducer may be used to introduce the tubular prosthesis 150 and create a telescoping interconnection. This second introducer may be based on the same principles as the introducer 100 described above, but less complex. For example, the second introducer may include a sheath for containing the tubular prosthesis 150 in a compressed state, so that it can be introduced into a targeted anatomy and then released to either self expand or be actively expanded with a balloon.

The second introducer may also be adapted so that it can introduce the tubular prosthesis 150 by passing it through one ostium in the bifurcated prosthesis 120 and partially out of another ostium until the terminus of the tubular prosthesis 150 that is closest to the external end of the second introducer is properly positioned. At that point, the tubular prosthesis 150 can be released from the second introducer.

### DEPLOYMENT

Prosthetic modules are preferably deployed seriatim. The modular interconnection between the tubular prosthesis 150 and the bifurcated prosthesis 120 is formed *in situ.* First the bifurcated prosthesis 120 is deployed, and then the tubular prosthesis 150 is deployed. For example, a bifurcated aortic prosthesis 120, as described in WO 98/53761, can be deployed into the abdominal aorta. The bifurcated prosthesis 120 has a generally inverted Y-shaped configuration having a body portion 123, a shorter leg 160 and a longer leg 132. The body of the prosthesis is constructed from a woven polyester tube. At the proximal end of the prosthesis 120 is a self-expanding stent 121 which extends beyond the end of the prosthesis and has distally extending barbs 151. The shorter leg 160 has projections on its distal terminus.

This bifurcated prosthesis 120 can be deployed in any method known in the art, preferably the method described in WO 98/53761 in which the devise is inserted by an introducer via a surgical cut-down into a femoral artery, and then advanced into the desired position over a stiff wire guide using endoluminal interventional techniques. For example, a guide wire (not shown) is first introduced into a femoral artery of the patient and advanced until its tip is beyond the desired deployment region of the prosthesis 120. At this stage, the introducer assembly 100 is fully assembled, and ready for introduction into the patient. The prosthesis 120 is retained at one end by the cylindrical sleeve 110 and the other by the distal attachment sections 140, and compressed by the sheath 130. If an aortic aneurism is to be repaired, the introducer assembly 100 can be inserted through a femoral artery over the guide wire, and positioned by radiographic techniques, which are not discussed here.

Once the introducer assembly 100 is in the desired deployment position, the sheath 130 is withdrawn to just proximal of the distal attachment section 140.
This action releases the middle portion of the prosthesis 120 so that it can expand radially. The proximal self-expanding stent 121, however, is still retained within the cylindrical sleeve 110. Also, the distal end 142 of the prosthesis 120 is still retained within the external sheath 130.

Next, the pin vise 139 is released to allow small movements of the thin walled tube 115 with respect to the thick walled tube 141. These movements allow the prosthesis 120 to be lengthened or shortened or rotated or compressed for accurate placement in the desired location within the lumen. X-ray opaque markers (not shown) may be placed along the prosthesis 120 to assist with placement of the prosthesis.

When the proximal end of the prosthesis 120 is in place, the proximal trigger wire is withdrawn by distal movement of the proximal wire release mechanism 124. The proximal wire release mechanism 124 and the proximal trigger wire can be completely removed by passing the proximal wire release mechanism 124 over the pin vise 139, the screw cap 146, and the connection means 116.

Next, the screw cap 146 of the pin vise 139 is then loosened. After this loosening, the thin walled tube 115 can be pushed in a proximal direction to move the cylindrical sleeve 110 in a proximal direction. When the cylindrical sleeve 110 no longer surrounds the self-expanding stent 121, the self-expanding stent 121 expands. When the self-expanding stent 121 expands, the barbs 151 grip the walls of the lumen to hold the proximal end of the prosthesis 120 in place. From this stage on, the proximal end of the prosthesis 120 typically cannot be moved.

Once the proximal end of the prosthesis 120 is anchored, the external sheath 130 is withdrawn to distal of the distal attachment section 140. This withdrawal allows the contralateral limb 160 and the longer leg 132 of the prosthesis 120 to expand. At this point, the distal end 142 of the prosthesis 120 may still be moved. Consequently, the prosthesis 120 can still be rotated or lengthened or shortened for accurate positioning. Such positioning of the prosthesis 120 may ensure that the shorter leg 160 extends in the direction of, and/or into a contralateral artery.

After the shorter leg 160 is deployed, the tubular prosthesis 150 can be deployed. The tubular prosthesis 150 is deployed such that it forms a telescoping interconnection with the shorter leg 160 such that it extends from the shorter leg 160 into the contralateral artery. The interconnection of the prosthesis 120 and tubular prosthesis 150, especially the formation of the engagement region, is described in greater detail in other portions of this disclosure.

The method of introduction of the second prosthetic module 150 is as follows. A guide wire (not shown) is introduced into the contralateral femoral artery and advanced until its tip is above the region into which the prosthesis is to be deployed.

The introducer is then advanced over the guide wire with an oscillating and rotating action until preferably about one stent of the extension prosthesis 150 is within the contralateral limb 160. A final position check may then be made before the sheath is withdrawn while holding the thick walled tube in place. The introducer can then be removed after the gap between the sheath and flexible extension is closed.

The release of the distal attachment device which contains the ipsilateral leg 132 and the withdrawal of the first introducer 100 can then proceed. A leg extension can then be attached to the ipsilateral leg, if indicated.

It may be preferable to introduce a second prosthetic module by passing one end of it through a portion of the first prosthetic module until the other end of the second prosthetic module is properly positioned to form a telescoping connection. At that point, the second prosthetic module can be allowed to expand, or actively balloon expanded. For example, if a bifurcated aortic prosthesis that Has branches for the renal arteries is placed into the aorta, a renal branch extension module can be introduced through an iliac artery, into the iliac leg of the bifurcated prosthesis and through the inside of the prosthesis. Then the renal branch extension can be deployed by passing it through the prosthetic branch until an appropriate length of the renal branch extension is within the prosthetic branch. At this point the renal branch extension can be allowed to expand or actively expanded to form a telescoping connection with the aortic prosthesis.

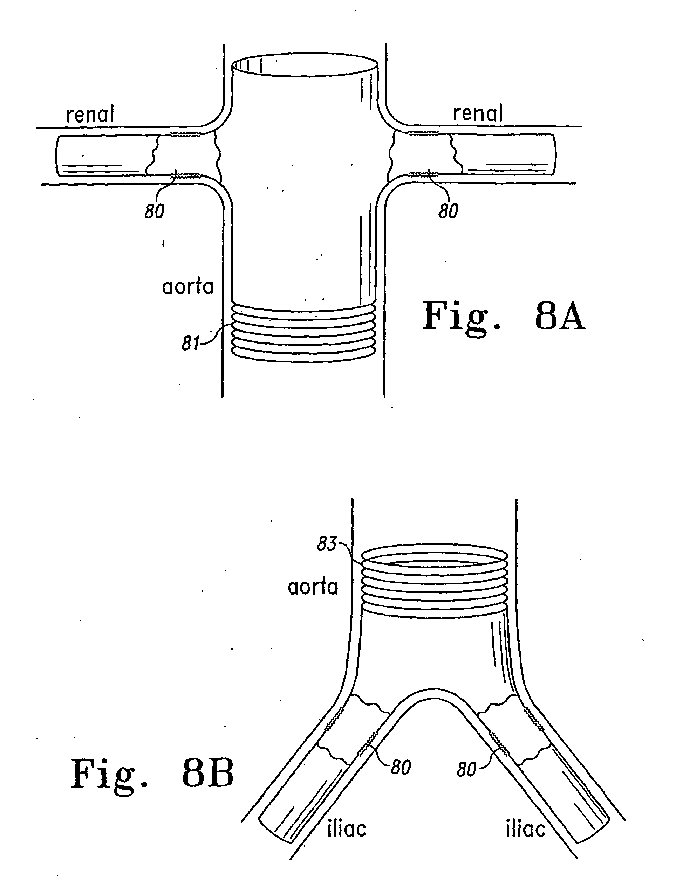

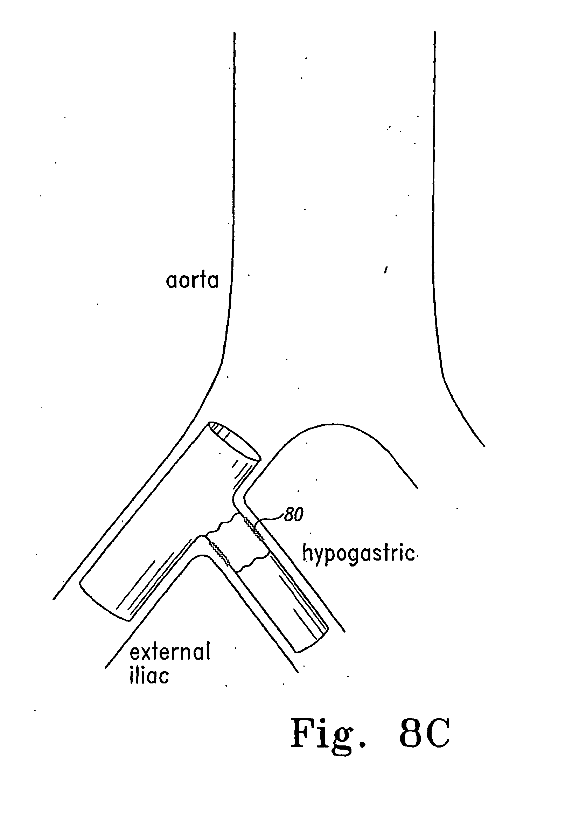

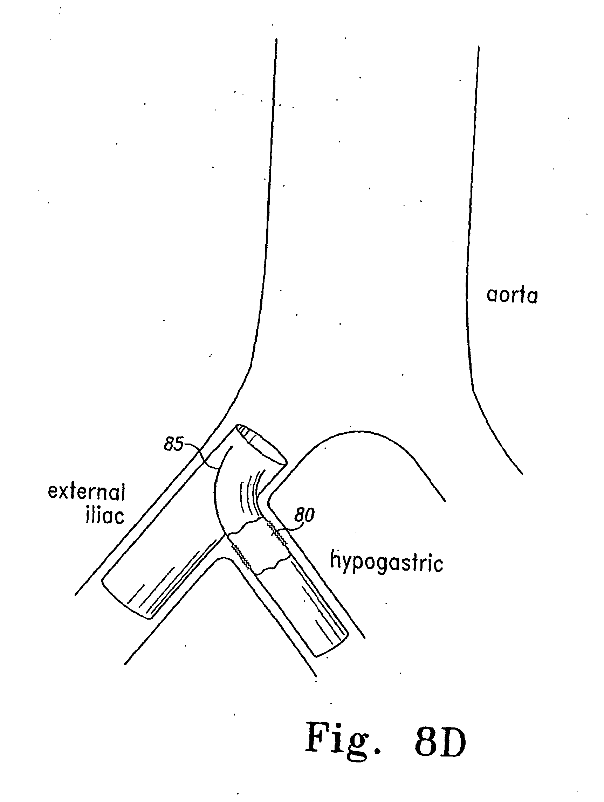

The introducer and deployment method described above can be adapted for implantation of prostheses in other regions. For example, if a first prosthetic module is placed into the aorta, an interconnecting prosthetic module can be placed into the renal (Figure 8A), iliac (Figure 8B), superior mesenteric, celiac or other artery to form a telescoping interconnection 80. Figures 8A also shows a crimped portion of the prosthetic trunk 81 which is interconnectable with the crimped portion of the prosthetic trunk 83 shown on Figure 8B. As shown in Figure 8C, if a first prosthetic module is placed into the iliac artery, an interconnecting prosthetic module can be placed into the hypogastric artery so that they form a telescoping interconnection 80. As shown in Figure 8D, if a first prosthetic module having a helical branch 85 is placed into the iliac artery, an interconnecting prosthetic module can be placed into the hypogastric artery, so that they form a telescoping interconnection 80. Helical prosthetic branches are further described in U.S. Application Publication No. 2004/0193254, filed January 13,200 and published September 30, 2004, entitled "Branched Vessel Endoluminal Device."

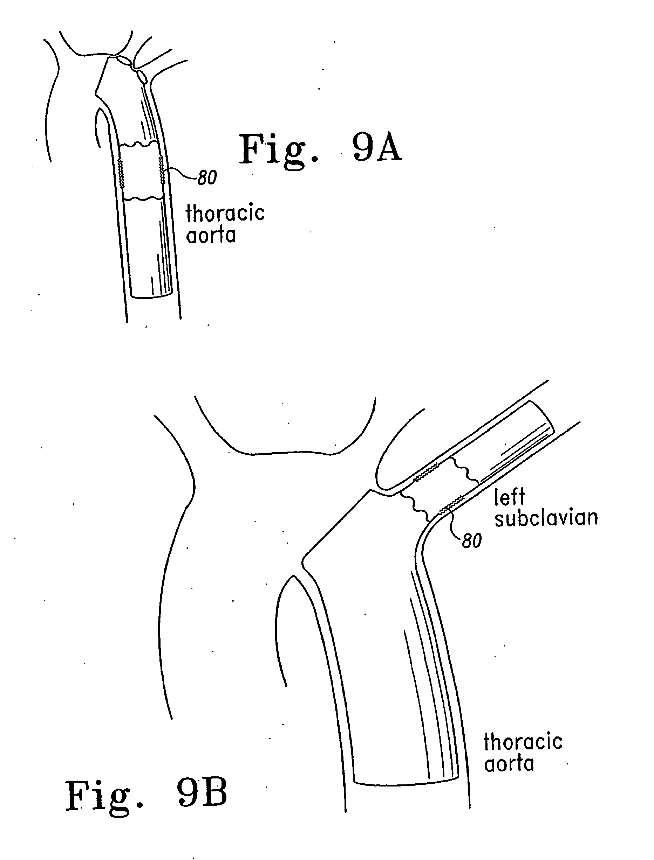

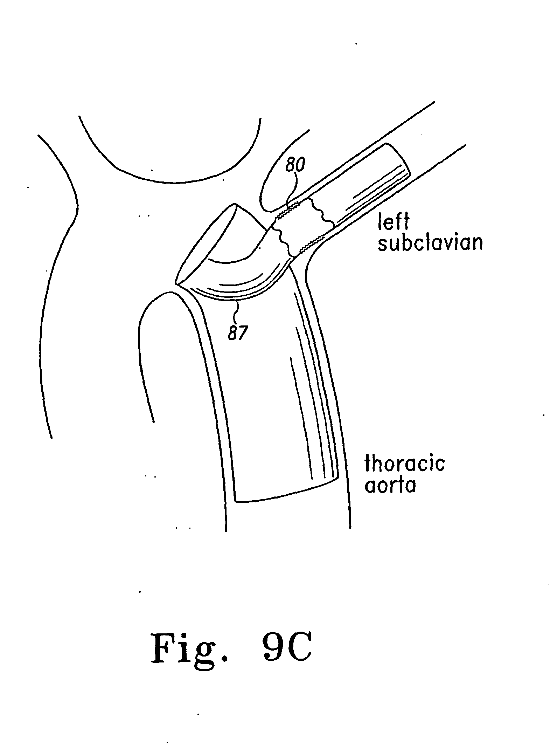

If a first prosthetic module is placed into the thoracic aorta, a connecting prosthetic module can be placed into another portion of the thoracic aorta (Figure 9A), the left subclavian (Figure 9B), left common carotid, innominate or other artery. As shown in Figure 9C, a thoracic aortic module may have a helical branch 87 that extends towards the left subclavian artery to interconnect with a left subclavian extension module at a telescoping interconnection 80. Furthermore, prosthetic modules which are implanted in the same artery can be connected to each other (Figure 8D). The overlap region 80 of each of these embodiments is preferably adapted to the size of the relevant anatomy and the forces to which the prostheses are exposed in the relevant anatomy.

Throughout this specification various indications have been given as to the scope of the invention but the invention is not limited to any one of these but may reside at two or more of these combined together. It is therefore intended that the foregoing detailed description be regarded as illustrative rather than limiting, and that it be understood that it is the following claims, including all equivalents, that are intended to define the scope of this invention.

Throughout this specification unless the context requires otherwise the words comprise and include and variations such as comprising and including will be understood to imply the inclusion of stated integers or group of integers but not the exclusion of any other integer or group of integers.

## Claims

1. A modular endoluminal prosthesis, comprising:
a first prosthetic module (40) having an opening at one end (41) and an internal surface (46); and
a second prosthetic module (50) having an opening at one end (43) and an external surface (48);
wherein the first and second modules (40, 50) are formed from a graft material and are connectable by inserting the one end (43) of the second module (50) into the one end (41) of the first module (40), wherein either the internal surface (46) or the external surface (48) comprises at least one projection (42 or 44) and the other of the internal surface (46) or the external surface (48) comprises at least one surface feature (42 or 44) that is formed in the graft material, said projection (42 or 44) and said surface feature (42 or 44) being at least partially complementary so that the surface feature (42 or 44) engages the projection (42 or 44) when the modules are connected.

2. The prosthesis of claim 1, wherein the at least one projection (42 or 44) is a first ridge (42 or 44).

3. The prosthesis of claim 2, wherein the at least one surface feature (42 or 44) is a second ridge (42 or 44), and wherein the first ridge (42 or 44) at least substantially complements the second ridge (42 or 44).

4. The prosthesis of claim 3, wherein the at least one projection (42 or 44) is a first helical ridge (42 or 44) and wherein the at least one surface feature (42 or 44) is a second helical ridge (42 or 44).

5. The prosthesis of claim 4, wherein the first helical ridge (42 or 44) and the second helical ridge (42 or 44) each having a pitch of at least about 1 mm.

6. The prosthesis of claim 1, wherein the at least one projection is a first plurality of substantially parallel helical ridges (34) and the at least one surface feature is a second plurality of substantially parallel helical ridges, wherein the first plurality of substantially parallel helical ridges (34) at least substantially complements the second plurality of substantially parallel helical ridges.

7. The prosthesis of claim 1, wherein the at least one projection is a first plurality of substantially parallel annular ridges (14).

8. The prosthesis of claim 7, wherein the at least one surface feature is a second plurality of substantially parallel annular ridges (14).

9. The prosthesis of claim 8, wherein the first plurality of substantially parallel annular ridges (14) at least substantially complements the second plurality of substantially parallel annular rides (14).

10. The prosthesis of claim 8 or 9, wherein the annular ridges of both the first plurality of substantially parallel annular ridges (14) and the second plurality of substantially parallel annular ridges (14) are spaced so as to have at least about 4 parallel ridges per centimeter.

11. The prosthesis of claim 8 or 9, wherein the annular ridges of both the first plurality of substantially parallel annular ridges (14) and the second plurality of substantially parallel annular ridges (14) are spaced so as to have at least about 8 parallel ridges per centimeter.

12. The prosthesis of claim 8 or 9, wherein the annular ridges of both the first plurality of substantially parallel annular ridges (14) and the second plurality of substantially parallel annular ridges (14) have a height of at least about 0.1mm.

13. The prosthesis of claim 1, 2, 6, 8 or 9, wherein the first prosthetic module is a bifurcated prosthetic module (83) comprising first and second legs and
wherein the first leg comprises the one end of the first prosthetic module, and
wherein the second prosthetic module is an extension module sized to form a telescoping connection with the first leg.

14. The prosthesis of claim 1, 2, 6, 7, 8 or 9, further comprising at least a first stent (68 or 70) disposed one of externally on the first prosthetic module (40) and internally on the first prosthetic module (40).

15. The prosthesis of claim 14, further comprising at least a second stent (68 or 70) disposed one of internally on the second prosthetic module (50) and externally on the second prosthetic module (50).

16. The prosthesis of claim 1, wherein both the at least one projection and the at least one surface feature comprises crimps.

17. The prosthesis of claim 1, wherein both the at least one projection and the at least one surface feature comprises corrugations.

18. The prosthesis of claim 1, 2, 6, 7, 8, 9, 16 or 17, wherein the at least one projection is formed in the graft material.

19. The prosthesis of claim 1, 2, 6, 7, 8, 9, 16 or 17, wherein a telescoping connection between the first prosthetic module (40) and the second prosthetic module (50) is capable of resisting a pull-out force of greater than 4 Newtons when the prosthesis is pressurized to 60mmHg and immersed in 37°C water.

20. The prosthesis of claim 1, 2, 6, 7, 8, 9, 16 or 17, wherein the telescoping connection between the first prosthetic module (40) and the second prosthetic module (50) is capable of resisting a pull-out force of greater than 7 Newtons when the prosthesis is pressurized to 60mmHg and immersed in 37°C water.

## Patentansprüche

1. Modulare endoluminale Prothese, die Folgendes umfasst:
ein erstes Prothesenmodul (40) mit einer Öffnung an einem Ende (41) und einer Innenfläche (46); und
ein zweites Prothesenmodul (50) mit einer Öffnung an einem Ende (43) und einer Außenfläche (48);
wobei das erste und das zweite Modul (40, 50) aus einem Graftmaterial gebildet sind und durch Einsetzen eines Endes (43) des zweiten Moduls (50) in das andere Ende (41) des ersten Moduls (40) verbunden werden können, wobei entweder die Innenfläche (46) oder die Außenfläche (48) mindestens einen Vorsprung (42 oder 44) umfasst und die jeweils andere Fläche, die Innenfläche (46) oder die Außenfläche (48) mindestens ein Flächenmerkmal (42 oder 44) umfasst, das in dem Graftmaterial ausgebildet ist, wobei der Vorsprung (42 oder 44) und das Flächenmerkmal (42 oder 44) zumindest teilweise komplementär sind, so dass das Flächenmerkmal (42 oder 44) den Vorsprung (42 oder 44) in Eingriff nimmt, wenn die Module verbunden sind.

2. Prothese nach Anspruch 1, wobei der mindestens eine Vorsprung (42 oder 44) ein erster Steg (42 oder 44) ist.

3. Prothese nach Anspruch 2, wobei das mindestens eine Flächenmerkmal (42 oder 44) ein zweiter Steg (42 oder 44) ist und wobei der erste Steg (42 oder 44) zu dem zweiten Steg (42 oder 44) zumindest im Wesentlichen komplementär ist.

4. Prothese nach Anspruch 3, wobei der mindestens eine Vorsprung (42 oder 44) ein erster schraubenförmiger Steg (42 oder 44) ist und wobei das mindestens eine Flächenmerkmal (42 oder 44) ein zweiter schraubenförmiger Steg (42 oder 44) ist.

5. Prothese nach Anspruch 4, wobei der erste schraubenförmige Steg (42 oder 44) und der zweite schraubenförmige Steg (42 oder 44) jeweils eine Steigung von mindestens ca. 1 mm aufweisen.

6. Prothese nach Anspruch 1, wobei der mindestens eine Vorsprung mehrere erste im Wesentlichen parallele schraubenförmige Stege (34) sind und das mindestens eine Oberflächenmerkmal mehrere zweite im Wesentlichen parallele schraubenförmige Stege sind, wobei die mehreren ersten im Wesentlichen parallelen schraubenförmigen Stege (34) zu den mehreren zweiten im Wesentlichen parallelen schraubenförmigen Stegen zumindest im Wesentlichen komplementär sind.

7. Prothese nach Anspruch 1, wobei der mindestens eine Vorsprung mehrere erste im Wesentlichen ringförmige Stege 14 sind.

8. Prothese nach Anspruch 7, wobei das mindestens eine Flächenmerkmal mehrere zweite im Wesentlichen parallele ringförmige Stege (14) sind.

9. Prothese nach Anspruch 8, wobei die mehreren ersten im Wesentlichen parallelen ringförmigen Stege (14) zu den mehreren zweiten im Wesentlichen parallelen ringförmigen Stegen (14) zumindest im Wesentlichen komplementär sind.

10. Prothese nach Anspruch 8 oder 9, wobei die ringförmigen Stege sowohl der mehreren ersten im Wesentlichen parallelen ringförmigen Stege (14) und der mehreren zweiten im Wesentlichen parallelen ringförmigen Stege (14) so voneinander beabstandet sind, dass sie mindestens ungefähr 4 parallele Stege pro Zentimeter aufweisen.

11. Prothese nach Anspruch 8 oder 9, wobei die ringförmigen Stege sowohl der mehreren ersten im Wesentlichen parallelen ringförmigen Stege (14) und die mehreren zweiten im Wesentlichen parallelen ringförmigen Stege (14) so voneinander beabstandet sind, dass sie mindestens ungefähr 8 parallele Stege pro Zentimeter aufweisen.

12. Prothese nach Anspruch 8 oder 9, wobei die ringförmigen Stege sowohl der mehreren ersten im Wesentlichen parallelen ringförmigen Stege (14) und die mehreren zweiten im Wesentlichen parallelen ringförmigen Stege (14) eine Höhe von mindestens ungefähr 0,1 mm aufweisen.

13. Prothese nach Anspruch 1, 2, 6, 8 oder 9, wobei das erste Prothesenmodul ein gegabeltes Prothesenmodul (83) ist, das einen ersten und einen zweiten Schenkel aufweist, und wobei der erste Schenkel das eine Ende des ersten Prothesenmoduls umfasst und wobei das zweite Prothesenmodul ein Verlängerungsmodul ist, das so bemessen ist, dass es eine teleskopische Verbindung mit dem ersten Schenkel bildet.

14. Prothese nach Anspruch 1, 2, 6, 7, 8 oder 9, die weiterhin mindestens einen ersten Stent (68 oder 70) umfasst, der entweder extern an dem ersten Prothesenmodul (40) oder intern an dem ersten Prothesenmodul (40) angeordnet ist.

15. Prothese nach Anspruch 14, die weiterhin mindestens einen zweiten Stent (68 oder 70) umfasst, der entweder intern an dem zweiten Prothesenmodul (50) oder extern an dem zweiten Prothesenmodul (50) angeordnet ist.

16. Prothese nach Anspruch 1, wobei sowohl der mindestens eine Vorsprung als auch das mindestens eine Flächenmerkmal Sicken umfasst.

17. Prothese nach Anspruch 1, wobei sowohl der mindestens eine Vorsprung als auch das mindestens eine Flächenmerkmal Wellungen umfasst.

18. Prothese nach Anspruch 1, 2, 6, 7, 8, 9, 16 oder 17, wobei der mindestens eine Vorsprung im Graftmaterial ausgebildet ist.

19. Prothese nach Anspruch 1, 2, 6, 7, 8, 9, 16 oder 17, wobei eine teleskopische Verbindung zwischen dem ersten Prothesenmodul (40) und dem zweiten Prothesenmodul (50) einer Herausziehkraft von über 4 Newton widerstehen kann, wenn die Prothese mit einem Druck von 60 mmHg beaufschlagt und in Wasser von 37°C eingetaucht wird.

20. Prothese nach Anspruch 1, 2, 6, 7, 8, 9, 16 oder 17, wobei die teleskopische Verbindung zwischen dem ersten Prothesenmodul (40) und dem zweiten Prothesenmodul (50) einer Herausziehkraft von über 7 Newton widerstehen kann, wenn die Prothese mit einem Druck von 60 mmHg beaufschlagt und in Wasser von 37°C eingetaucht wird.

## Revendications

1. Prothèse endoluminale modulaire, comprenant :
un premier module prothétique (40) ayant une ouverture à une extrémité (41) et une surface interne (46) ; et
un deuxième module prothétique (50) ayant une ouverture à une extrémité (43) et une surface externe (48) ;
les premier et deuxième modules (40, 50) étant formés d'un matériau de greffe et pouvant être connectés en insérant la première extrémité (43) du deuxième module (50) dans la première extrémité (41) du premier module (40), la surface interne (46) ou la surface externe (48) comprenant au moins une saillie (42 ou 44) et l'autre surface, la surface interne (46) ou la surface externe (48) comprenant au moins une structure de surface (42, ou 44) formée dans le matériau de greffe, ladite saillie (42 ou 44) et ladite structure de surface (42 ou 44) étant au moins en partie complémentaires de sorte que la structure de surface (42 ou 44) s'engage avec la saillie (42 ou 44) lorsque les modules sont connectés.

2. Prothèse selon la revendication 1, dans laquelle l'au moins une saillie (42 ou 44) est une première crête (42 ou 44).

3. Prothèse selon la revendication 2, dans laquelle l'au moins une structure de surface (42 ou 44) est une deuxième crête (42 ou 44) et dans laquelle la première crête (42 ou 44) est au moins substantiellement complémentaire de la deuxième crête (42 ou 44).

4. Prothèse selon la revendication 3, dans laquelle l'au moins une saillie (42 ou 44) est une première crête hélicoïdale (42 ou 44) et dans laquelle l'au moins une structure de surface (42 ou 44) est une deuxième crête hélicoïdale (42 ou 44).

5. Prothèse selon la revendication 4, dans laquelle la première crête hélicoïdale (42 ou 44) et la deuxième crête hélicoïdale (42 ou 44) ont chacune un pas d'au moins 1 mm.

6. Prothèse selon la revendication 1, dans laquelle l'au moins une saillie est une première pluralité de crêtes hélicoïdales substantiellement parallèles (34) et l'au moins une structure de surface est une deuxième pluralité de crêtes hélicoïdales substantiellement parallèles, la première pluralité de crêtes hélicoïdales substantiellement parallèles (34) étant au moins substantiellement complémentaire de la deuxième pluralité de crêtes hélicoïdales substantiellement parallèles.

7. Prothèse selon la revendication 1, dans laquelle l'au moins une saillie est une première pluralité de crêtes annulaires substantiellement parallèles (14).

8. Prothèse selon la revendication 7, dans laquelle l'au moins une structure de surface est une deuxième pluralité de crêtes annulaires substantiellement parallèles (14).

9. Prothèse selon la revendication 8, dans laquelle la première pluralité de crêtes annulaires substantiellement parallèles (14) est au moins substantiellement complémentaire de la deuxième pluralité de crêtes annulaires substantiellement parallèles (14).

10. Prothèse selon la revendication 8 ou 9, dans laquelle les crêtes annulaires de la première pluralité de crêtes annulaires substantiellement parallèles (14) et de la deuxième pluralité de crêtes annulaires substantiellement parallèles (14) sont espacées de manière à avoir au moins environ 4 crêtes parallèles par centimètre.

11. Prothèse selon la revendication 8 ou 9, dans laquelle les crêtes annulaires de la première pluralité de crêtes annulaires substantiellement parallèles (14) et de la deuxième pluralité de crêtes annulaires substantiellement parallèles (14) sont espacées de manière à avoir au moins environ 8 crêtes parallèles par centimètre.

12. Prothèse selon la revendication 8 ou 9, dans laquelle les crêtes annulaires de la première pluralité de crêtes annulaires substantiellement parallèles (14) et de la deuxième pluralité de crêtes annulaires substantiellement parallèles (14) ont une hauteur d'au moins environ 0,1 mm.

13. Prothèse selon la revendication 1, 2, 6, 8 ou 9, dans laquelle le premier module prothétique est un module prothétique bifurqué (83) comprenant des première et deuxième pattes et dans laquelle la première patte comprend la première extrémité du premier module prothétique et dans laquelle le deuxième module prothétique est un module d'extension dimensionné de manière à former une connexion télescopique avec la première patte.

14. Prothèse selon la revendication 1, 2, 6, 7, 8 ou 9, comprenant en outre au moins un premier stent (68 ou 70) disposé soit à l'extérieur sur le premier module prothétique (40) soit à l'intérieur sur le premier module prothétique (40).

15. Prothèse selon la revendication 14, comprenant en outre au moins un deuxième stent (68 ou 70) disposé soit à l'intérieur sur le deuxième module prothétique (50) soit à l'extérieur sur le deuxième module prothétique (50).

16. Prothèse selon la revendication 1, dans laquelle l'au moins une saillie et l'au moins une structure de surface comprennent des contacts de sertissage.

17. Prothèse selon la revendication 1, dans laquelle l'au moins une saillie et l'au moins une structure de surface comprennent des ondulations.

18. Prothèse selon la revendication 1, 2, 6, 7, 8, 9, 16 ou 17, dans laquelle l'au moins une saillie est formée dans le matériau de greffe.

19. Prothèse selon la revendication 1, 2, 6, 7, 8, 9, 16 ou 17, dans laquelle une connexion de type télescopique entre le premier module prothétique (40) et le deuxième module prothétique (50) est capable de résister à une force d'extraction supérieure à 4 Newtons lorsque la prothèse est pressurisée à 60 mmHg et immergée dans de l'eau à 37°C.

20. Prothèse selon la revendication 1, 2, 6, 7, 8, 9, 16 ou 17, dans laquelle la connexion de type télescopique entre le premier module prothétique (40) et le deuxième module prothétique (50) est capable de résister à une force d'extraction supérieure à 7 Newtons lorsque la prothèse est pressurisée à 60 mmHg et immergée dans de l'eau à 37°C.
